(19) **Europäisches Patentamt / European Patent Office / Office européen des brevets**

(11) **EP 3 584 328 A1**

(12) ## EUROPEAN PATENT APPLICATION
published in accordance with Art. 153(4) EPC

(43) Date of publication:
**25.12.2019 Bulletin 2019/52**

(51) Int Cl.:
*C21C 5/46* (2006.01)    *G01N 21/17* (2006.01)
*G01N 33/20* (2019.01)

(21) Application number: **18754117.2**

(22) Date of filing: **13.02.2018**

(86) International application number:
**PCT/JP2018/004831**

(87) International publication number:
**WO 2018/151078 (23.08.2018 Gazette 2018/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **14.02.2017 JP 2017025440**

(71) Applicant: **Nippon Steel Corporation
Tokyo 1008071 (JP)**

(72) Inventors:
• **KUSUNOKI Tomoyuki**
  **Tokyo 100-8071 (JP)**
• **MIYAZAKI Takahiro**
  **Tokyo 100-8071 (JP)**

(74) Representative: **Vossius & Partner
Patentanwälte Rechtsanwälte mbB
Siebertstrasse 3
81675 München (DE)**

(54) **METHOD FOR DETECTING SLAG IN MOLTEN STEEL FLOW**

(57)    A method of detecting slag in a molten steel flow, includes a histogram creation step of creating a histogram for a captured image of a molten steel flow including molten steel and slag, a maximum peak point detection step of detecting a maximum peak point of the histogram, an intermediate peak point detection step of detecting an intermediate peak point of the histogram, an intermediate peak point counting step of counting the number $Nh$ of intermediate peak points having a density parameter larger than the density parameter at the maximum peak point and the number $Nl$ of intermediate peak points having a density parameter smaller than the density parameter at the maximum peak point, and a maximum peak point type determination step of determining a type of the maximum peak point by a magnitude relationship between the number $Nl$ and the number $Nh$.

FIG. 2

```
            START
              |
     IMAGE CAPTURING STEP ─── ST1
              |
     HISTOGRAM CREATION  ─── ST2
           STEP
              |
     MAXIMUM PEAK POINT  ─── ST3
      DETECTION STEP
              |
     INTERMEDIATE PEAK   ─── ST4
    POINT DETECTION STEP
              |
     INTERMEDIATE PEAK   ─── ST5
    POINT COUNTING STEP
              |
     MAXIMUM PEAK POINT  ─── ST6
     TYPE DETERMINATION
            STEP
       /              \
  Nh<Nl              Nh>Nl
MAXIMUM PEAK POINT   MAXIMUM PEAK POINT
  = SLAG              = MOLTEN STEEL
      |                  |
ST7 ─ FIRST          SECOND  ─ ST8
  DETERMINATION    DETERMINATION
     STEP              STEP
       \              /
            END
```

EP 3 584 328 A1

## Description

[Technical Field of the Invention]

The present invention relates to a method of detecting slag in a molten steel flow.

Priority is claimed on Japanese Patent Application No. 2017-025440, filed February 14, 2017, the content of which is incorporated herein by reference.

[Related Art]

When molten steel is tapped from a converter to a ladle, generally, the converter is tilted to cause a molten steel flow to be directed from the converter toward the ladle. In this case, it is ideal to leave slag in the converter and cause only the molten steel to flow from the converter to the ladle. However, generally, in the molten steel flow directed from the converter toward the ladle, substantially only the molten steel exists in an early stage of the tapping, but the molten steel and the slag are mixed with each other between a middle stage of the tapping and a last stage of the tapping. Accordingly, when trying to prevent an outflow of the slag, the molten steel remains in the converter, and thus, there is a concern that a yield may become low.

Meanwhile, when trying to reduce the residual amount of the molten steel in the converter, the slag flows toward the ladle together with the molten steel, and thus, a large amount of slag exists in the ladle. As a result, there are problems that blowout of the slag from the ladle occurs or component deviation of the molten steel occurs in a secondary refining step which is a later step.

Accordingly, it is desirable to detect the slag in the molten steel flow directed from the converter toward the ladle, to quantify an amount of outflow of the slag, and to control the amount of outflow of the slag to a range required in a tapping operation of the converter.

An emissivity of the slag is higher than that of the molten steel, if an image of the molten steel flow is captured, a region in which the slag exists is image-captured brighter than a region in which the slag does not exist and only the molten steel exists. In other words, a density (gray level) of a pixel region corresponding the slag in a captured image obtained by capturing the image of the molten steel flow is larger than a density of a pixel region corresponding to the molten steel. For example, a technology of detecting the slag using this principle, there are methods described in Patent Document 1.

Patent Document 1 discloses a method of creating a density (brightness) histogram in which a density (brightness) of a captured image obtained by capturing an image of a molten steel flow is shown on a horizontal axis and the number of pixels thereof is shown on a vertical axis and detecting slag using this density histogram. Specifically, in the method of Patent Document 1, in the density histogram, a maximum peak point (maximum peak position) having a largest number of pixels is considered to correspond to the molten steel, a pixel having a density value (brightness value) of N1 or more considering a standard deviation $\sigma$ in a horizontal axis direction of the maximum peak point is determined as the molten steel, and a pixel having a density value (brightness value) of N2 or more obtained by adding a bias value B to the density value N1 is determined as the slag.

However, the present inventors examined, and thus, it turned out that the maximum peak point in the density histogram does not necessarily correspond to the molten steel, but may correspond to the slag. Therefore, it is difficult to accurately detect the slag by the method of Patent Document 1 which determines the density value N2, assuming that the maximum peak point always corresponds to the molten steel.

Here, in a case where one peak exist in the density histogram, using the emissivity of the slag and the emissivity of the molten steel being different with each other (the emissivity of the slag is higher than the emissivity of the molten steel), it is considered that it can be determined whether this peak corresponds to the molten steel or the slag. In addition, also in a case where two peaks having smooth curves exist in the density histogram, using the emissivity of the slag and the emissivity of the molten steel being different with each other, for example, it is considered that it can be determined the peak on a low temperature side corresponds to the molten steel and the peak on a high temperature corresponds to the slag.

However, in a case where a plurality of sub peaks are observed in a histogram of a captured image obtained by capturing an image of the molten steel flow in which the molten steel and the slag are mixed, it is difficult to use the above-described method, and there is a concern that accuracy of detecting the slag decreases.

In addition, for example, a temperature of the molten steel flow is changed to 100°C or more depending on a kind of steel or a condition of a tapping operation. Accordingly, when trying to perform a determination using a fixed threshold value, in a case where the temperature of the molten steel flow is changed, there is a concern that the accuracy of detecting the slag decreases.

[Prior Art Document]

[Patent Document]

[Patent Document 1] Japanese Unexamined Patent Application, First Publication No. 2006-213965

[Disclosure of the Invention]

[Problems to be Solved by the Invention]

The present invention is made in consideration of the above-described circumstances, and an object thereof is to provide a method of detecting slag in a molten steel flow capable of accurately detecting the slag in the molten steel flow in even a case where a temperature of the molten steel flow is changed.

[Means for Solving the Problem]

In order to solve the above-mentioned problem, the present inventors intensively studied.

First, the present inventors captured images of various molten steel flows over an early stage of tapping, a middle stage of the tapping, and a last stage of the tapping using an image capturing unit such as a thermal image camera (thermography) having a main sensitivity in an infrared light region, and thereby many captured images were obtained. In addition, for each of the captured images, a histogram was created in which a temperature was shown on a horizontal axis and the number of pixels was shown on a vertical axis. For example, in a temperature range of the horizontal axis of 1000° to 2000°C, the present inventors found that there might be a maximum peak point in which the number of the pixels of the vertical axis was a maximum value, and if the maximum peak point might be positioned on a low temperature side, the maximum peak point might be positioned on a high temperature side.

Next, the present inventors found that, in a case where slag did not exist in the molten steel flow or in a case where a very small amount of slag existed in the molten steel flow, in the histogram, the maximum peak point corresponded to the molten steel and was positioned on a low temperature side, and in a case where a large amount of slag did exist in the molten steel flow, in the histogram, the maximum peak point corresponded to the slag and was positioned on a high temperature side. However, as described above, a temperature of the molten steel flow is changed. Accordingly, in a case where it is determined to which of the molten steel and the slag the maximum peak point corresponds by determining on which of the low temperature side and the high temperature side of a fixed threshold value the maximum peak point is positioned using the fixed threshold value for the temperature, it is difficult to accurately detect the slag.

Therefore, the present inventors more intensively studied. The present inventors noted that, in the histogram at the captured image of the molten steel flow including the molten steel and the slag, in addition to the maximum peak point, a peak point (hereinafter, referred to as an "intermediate peak point") which has the number of pixels less than the number of pixels of the maximum peak point and is local maximum value equal to or more than a predetermined threshold value (for example, 50% of the number of pixels of the maximum peak point) of the number of pixels might exist. In this case, for example, the present inventors found that, even when the temperature of the molten steel flow is changed depending on the kind of steel or the condition of the tapping operation, in a case where the maximum peak point corresponded to the molten steel, the number of intermediate peak points having a temperature higher than a temperature at the maximum peak point was greater than the number of intermediate peak points having a temperature lower than the temperature at the maximum peak point.

In addition, the present inventors found that, even when the temperature of the molten steel flow is changed depending on the kind of steel or the condition of the tapping operation, in a case where the maximum peak point corresponded to the slag, the number of intermediate peak points having a temperature lower than the temperature at the maximum peak point was greater than the number of intermediate peak points having a temperature higher than the temperature at the maximum peak point.

In addition, the temperature is mentioned as an example as described above. However, the present inventors found that the same could be applied a histogram in which a density before being converted into a temperature was shown on a horizontal axis. In addition, the present inventors found that the same could be applied to a histogram in which a density was shown on a horizontal axis and the number of pixel was shown a vertical axis for a captured image obtained by capturing an image of a molten steel flow using a CCD camera having a main sensitivity in a visible light region.

Based on the above findings, the present invention adopts the following in order to solve the above problems.

(1) According to an aspect of the present invention, there is provided a method of detecting slag in a molten steel flow, including: an image capturing step of sequentially capturing a molten steel flow which is directed from a converter toward a ladle and includes molten steel and slag to acquire a captured image of the molten steel flow; a histogram creation step of creating, by performing image processing on the captured image, a histogram in which a density parameter corresponding to a density of each pixel constituting the captured image is shown on a horizontal axis and the number of pixels that is a total number of pixels each having the density parameter is shown on a vertical axis; a maximum peak point detection step of detecting a maximum peak point, in which the number of pixels is an absolute maximum value, for the histogram; an intermediate peak point detection step of detecting an intermediate peak point which has the number of pixels less than the number of pixels of the maximum peak point and is a local maximum value equal to or more than a predetermined threshold value of the number of pixels, for the histogram; an intermediate peak point counting step of counting the number $Nh$ of intermediate peak points having a density parameter larger than the density parameter at the maximum peak point and the number $N1$ of intermediate peak points having a density parameter smaller than the density parameter at the maximum peak point; and a maximum peak point type determination step of determining that the maximum peak point corresponds to the molten steel in a case where the number Nh is larger than the number N1 while determining that the maximum peak point corresponds to the slag in a case where the number N1 is larger than the number Nh.

(2) In the aspect described in (1), the following configuration may be adopted: the method further include a first determination step of, in a case where it is determined that the maximum peak point corresponds to the slag in the maximum peak point type determination step, determining that a pixel having a density parameter less than a first threshold value determined based on the maximum peak point corresponds to the molten steel and a pixel having a density parameter equal to or more than the first threshold value corresponds to the slag; and

a second determination step of, in a case where it is determined that the maximum peak point corresponds to the molten steel in the maximum peak point type determination step, determining that a pixel having a density parameter equal to or less than a second threshold value determined based on the maximum peak point corresponds to the molten steel and a pixel having a density parameter more than the second threshold value corresponds to the slag.

(3) In the aspect described in (2), the following configuration may be adopted: the first threshold value is represented by a first straight line, which passes through the maximum peak point and has a positive inclination, in the histogram, the second threshold value is represented by a second straight line, which passes through the maximum peak point and has a negative inclination, in the histogram, and an absolute value of an inclination of the second straight line is larger than an absolute value of an inclination of the first straight line.

(4) In the aspect described in (3), the first straight line is a straight line which passes through a peak point having a maximum density parameter from among points having the number of pixels less than a threshold value of the number of pixels and a density parameter smaller by a predetermined value or more with respect to the density parameter at the maximum peak point, and the maximum peak point, and the absolute value of the inclination of the second straight line is 1.5 to 2.5 times the absolute value of the inclination of the first straight line.

[Effects of the Invention]

According to each aspect of the present invention, it is possible to accurately detect slag in a molten steel flow in even a case where a temperature of the molten steel flow is changed.

[Brief Description of the Drawings]

FIG. 1 is a schematic view showing a schematic configuration of a slag detection device used in a slag detection method according to an embodiment of the present invention.
FIG. 2 is a flowchart showing a schematic procedure of the slag detection method.
FIG. 3A is a view showing an example of a captured image acquired in an image capturing step ST1 shown in FIG. 2.
FIG. 3B is a graph showing a histogram created based on the captured image of FIG. 3A in a histogram creation step ST2 shown in FIG. 2.
FIG. 4 is a graph for explaining a first threshold value determined in a first determination step ST7 shown in FIG. 2.
FIG. 5A is a view showing an example of the captured image acquired in the image capturing step ST1 shown in FIG. 2 and is a view showing an example different from that of FIG. 3A.
FIG. 5B is a graph showing a histogram created based on the captured image of FIG. 5A in the histogram creation step ST2 shown in FIG. 2.
FIG. 6 is a graph for explaining a second threshold value determined in a second determination step ST8 shown in FIG. 2.
FIG. 7A is a graph for explaining a slag detection method described in Patent Document 1.
FIG. 7B is a view showing a captured image used to create the histogram of FIG. 7A.
FIG. 8 is a view showing a result of extracting a pixel region in which slag S exists in the captured image shown in FIG. 3A by difference processing.

[Embodiments of the Invention]

Hereinafter, a method of detecting slag (hereinafter, simply referred to as a "slag detection method") in a molten steel flow according to an embodiment of the present invention will be described with reference to the drawings. In addition, in the present specification and drawings, the same reference symbol is assigned to the same component having substantially the same functional configuration, and repeated descriptions thereof are omitted.

First, a configuration of a slag detection device 100 used in the slag detection method according to the present embodiment will be described.

<Configuration of Slag Detection Device 100 of Present Embodiment>

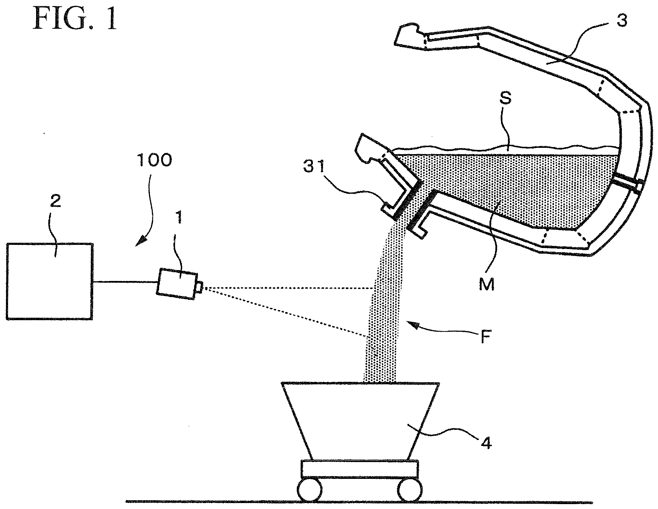

FIG. 1 is a schematic view showing a schematic configuration of the slag detection device 100. In addition, in FIG. 1, a converter 3 which accommodates molten steel M and slag S is shown in cross section.

As shown in FIG. 1, when tapping from the converter 3 to a ladle 4 is performed, the slag detection device 100 is used to detect the slag S in a molten steel flow F directed from a tapping hole 31 of the tilted converter 3 toward the ladle 4. The slag detection device 100 includes an image capturing unit 1 which approximately horizontally captures an image of the molten steel flow F approximately vertically directed from the tapping hole 31 of the converter 3 toward the ladle 4 and an image processor 2 which is connected to the image capturing unit 1.

For example, a thermal imaging camera (thermography) having a main sensitivity in an infrared light region, a CCD camera having a main sensitivity in a visible light region, or the like can be used as the image capturing unit 1. As these thermal imaging camera (thermography) and the CCD camera, for example, commercially available cameras can be used.

In the present embodiment, a thermal imaging camera having a main sensitivity in an infrared light region is used as the image capturing unit 1. In addition, in a case where the thermal imaging camera (thermography) is used like the present embodiment, a value of a temperature or density (density before converting into a temperature) of a pixel region in a captured image can be calculated. Meanwhile, in a case where the CCD camera is used, a value of a density of the pixel region can be calculated.

For example, the image processor 2 is constituted by a general-purpose personal computer in which a predetermined program is executed for a histogram creation step ST2 or the like described later is installed. In addition, the image processor 2 has a monitor for displaying the captured image obtained by the image capturing unit 1.

The slag detection method according to the present embodiment is performed using the slag detection device 100. Hereinafter, the slag detection method according to the present embodiment will be described.

<Slag Detection Method of Present Embodiment>

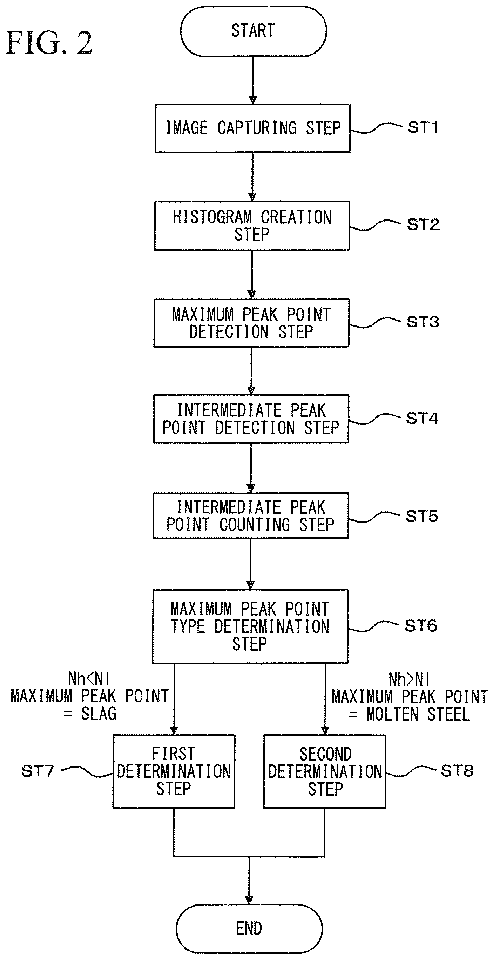

FIG. 2 is a flowchart showing a schematic procedure of the slag detection method according to the present embodiment.

The slag detection method according to the present embodiment is a method of detecting the slag S in the molten steel flow F based on an captured images obtained by capturing an image of the molten steel flow F, which is directed from the converter 3 toward the ladle 4 and includes the molten steel M and the slag S, by the image capturing unit 1. As shown in FIG. 2, the slag detection method has an image capturing step ST1, the histogram creation step ST2, a maximum peak point detection step ST3, an intermediate peak point detection step ST4, an intermediate peak counting step ST5, a maximum peak point type determination step ST6, a first determination step ST7, and a second determination step ST8.

Hereinafter, a content of each step will be sequentially described.

(Imaging Capturing Step ST1)

In the image capturing step ST1, the image of the molten steel flow F directed from the converter 3 toward the ladle 4 is captured by the image capturing unit 1 so as to acquire the captured image (refer to FIG. 1).

In the present embodiment, the thermal imaging camera is used as the image capturing unit 1, and thus, a captured image acquired in the image capturing step ST1 is obtained by converting a density of each pixel constituting the captured image into a temperature by a predetermined conversion formula. That is, the captured image acquired in the image capturing step ST1 has a value of a temperature detected for each pixel.

A visual field of the image capturing unit 1 is set to a wide visual field including not only the molten steel flow F but also a background so as not to be affected by fluctuations of an outflow position and spread of the molten steel flow F. Even when the visual field of the image capturing unit 1 is set to include the background, a temperature of the background is lower than a temperature of the molten steel flow F, and thus, in the maximum peak point detection step ST3 described later, the pixel region corresponding to the molten steel flow F and the pixel region corresponding to the background can be identified. In addition, the visual field of the image capturing unit 1 may be adjusted narrowly in advance such that only the image of the molten steel flow F is captured. However, in general, the outflow position and the spread of the molten steel flow F fluctuate to some extent depending on a tilt angle of the converter 3 or the like (depending on a position of the tapping hole 31 or the like). For this reason, adjusting the visual field of the image capturing unit 1 such that only the image of the molten steel flow F is captured in any of an early stage of tapping, a middle stage of the tapping, and a last stage of the tapping is a laborious task. Accordingly, it is preferable that the visual field of the image capturing unit 1 is set to a wide visual field including the background.

An image capturing timing of the image capturing unit 1 is not particularly limited. However, in order to increase a time resolution for detecting the slag S, it is preferable to continuously capture an image for each scanning period (a reciprocal number of a frame rate) set in the image capturing unit 1.

The captured image obtained by the image capturing unit 1 is stored in the image processor 2.

(Histogram Creation Step ST2)

In the histogram creation step ST2, the image processor 2 performs image processing on the captured image acquired in the image capturing step ST1, and a histogram is created, in which a density parameter corresponding to a density of each pixel constituting the captured image is shown on a horizontal axis and the number of pixels which is a total number of pixels each having this density parameter is shown on a vertical axis. The histogram may be created for each captured image or may be created for an average image obtained by averaging a plurality of continuous captured images. In a case where the average image is used, it is preferable to average the plurality of captured images which are continuous within a time (= L/V) obtained by dividing a length L of a pixel region corresponding to the molten steel flow F in the visual field of the image capturing unit 1 by a velocity V of the molten steel flow F.

As the density parameter, a temperature can be exemplified in addition to the density. In a case where the image capturing unit 1 is a thermal imaging camera as in the present embodiment, it is possible to create a histogram whose horizontal axis is a temperature or a density (density before converting to a temperature). Meanwhile, in a case where the image capturing unit 1 is a CCD camera, it is possible to create a histogram whose horizontal axis is a density.

Here, for example, the "density" in the present specification refers to brightness and darkness (that is, the brightness on the image) of an image of 256 gradations. In addition, a relationship between this density and thermal radiation brightness in the molten steel flow is in a linear relationship.

As described above, since the thermal imaging camera is used as the image capturing unit 1 in the present embodiment, a temperature is used as the density parameter (that is, in the present embodiment, the horizontal axis of the histogram is a temperature).

In the present embodiment, as described above, the visual field of the image capturing unit 1 is set to include not only the molten steel flow F but also the background. Therefore, when the histogram is created, the image processor 2 determines that a pixel region having a temperature equal to or higher than a predetermined threshold value (for example, 1000°C) in the captured image is a pixel region corresponding to the molten steel flow F and create a histogram for this pixel region (that is, the histogram creation is not applied to a pixel region whose temperature, which is the horizontal axis, is less than the predetermined threshold value). Accordingly, it is possible to avoid an influence of the background on the histogram (the number of pixels corresponding to the background does not become an absolute maximum value).

In addition, the image processor 2 may create a histogram for all the captured images including the pixel regions corresponding to the background and may avoid the influence of the background by excluding a temperature less than the predetermined threshold value (for example, 1000°C) from a detection range of a maximum peak point in the maximum peak point detection step ST3 described later.

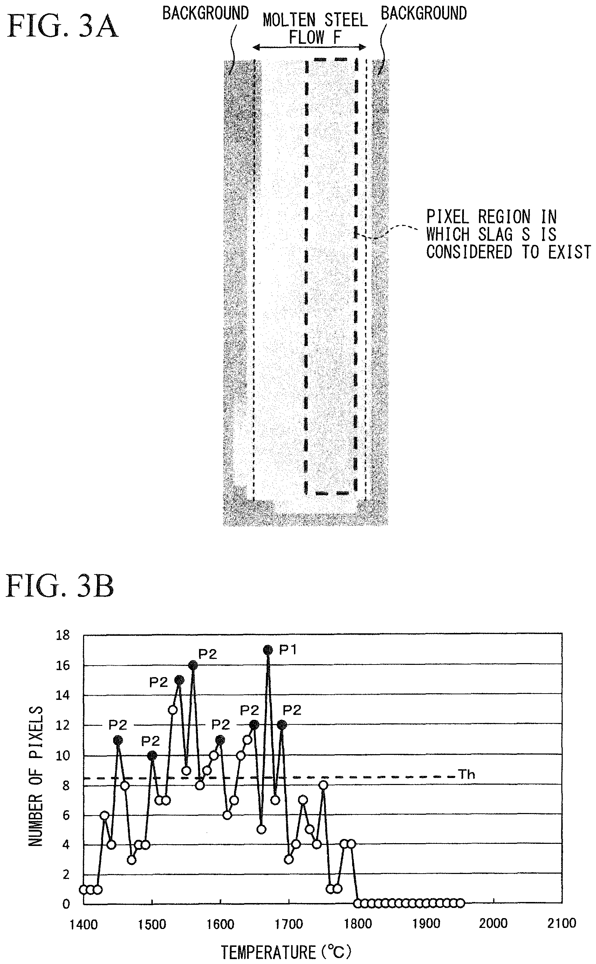

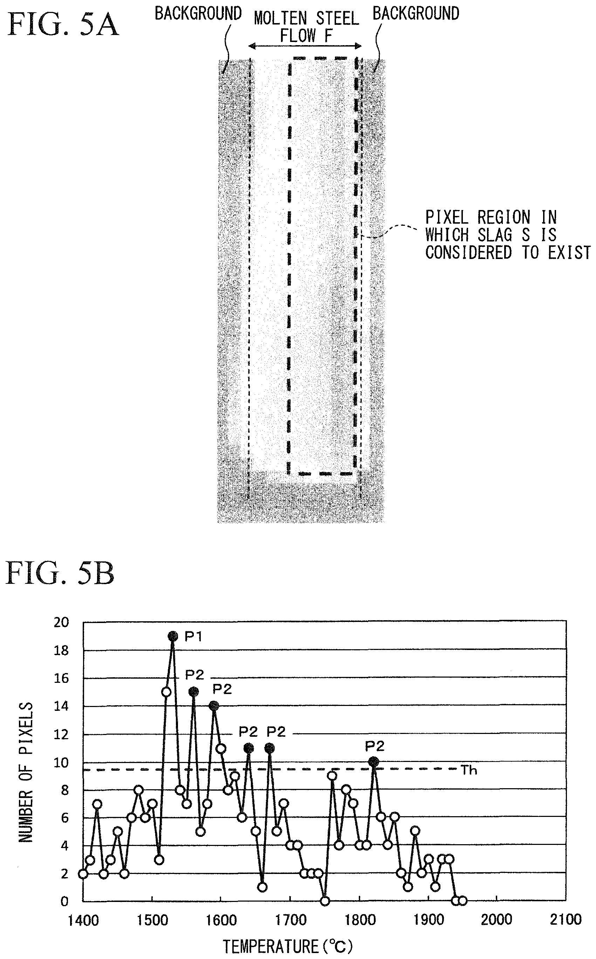

FIGS. 3A is a view showing an example of the captured image acquired in the image capturing step ST1. Specifically, for example, FIG. 3A is an example of an average image obtained by averaging five captured images continuously acquired for each scanning period of the image capturing unit 1 (the resolution of the captured image is about 3 cm/pixel). In FIG. 3A, only the pixel region corresponding to the molten steel flow F in the captured image (average image) and the pixel region corresponding to the background positioned in the vicinity thereof are partially cut out and displayed. That is, in the captured image to be actually acquired, the pixel region in a right-left direction on the paper surface is wider than in the captured image shown in FIGS. 3A and 5A.

In addition, although the captured image shown in FIG. 3A is displayed in monochrome for the convenience of illustration, in actual, different colors depending on a temperature of each pixel are added and displayed on the monitor included in the image processor 2. That is, since the temperature of the pixel region corresponding to the molten steel flow F is higher than the temperature of the pixel region corresponding to the background, in an actual captured image obtained in the image capturing step ST1, the color corresponding to the high temperature is colored.

Moreover, among pixel regions corresponding to the molten steel flow F, a temperature (apparent temperature) of a pixel region (specifically, a pixel region which is determined to correspond to the slag S in the first determination step ST7 described later) which is surround by thick dotted lines in FIG. 3A and in which the slag S is considered to exist is higher than temperatures (apparent temperatures) of the other pixel regions (regions of pixels in which substantially only the molten steel M exist), and a color corresponding to the high temperature is colored to the pixel region in which the slag S is considered to exist.

In addition, in the molten steel flow F discharged from the converter 3, it is considered that an actual temperature of the region corresponding to the pixel region in which the slag S exists and an actual temperature of the region corresponding to the pixel region in which substantially only the molten steel M exists are equivalent to each other. However, in general, an emissivity of the slag S is higher than an emissivity of the molten steel M (the emissivity of the slag is about 1.5 times the emissivity of the molten steel) and the emissivity in the image capturing unit 1 is set to be the same for any pixel. Accordingly, as described above, the obtained captured image, the temperature of the pixel region in which the slag S exists is measured higher than the temperature of the pixel region in which substantially only the molten steel M exists. The same is applied to FIG. 5A described later.

FIG. 3B is a graph showing a histogram created for the captured image (average image) shown in FIG. 3A. When the histogram of FIG. 3B is created, in order to avoid the influence of the background, a temperature range of the horizontal axis is set to the predetermined threshold value (1000°C) or more (However, illustration is omitted for temperatures below 1400°C, where no feature is found in a distribution of the number of pixels), the horizontal axis is divided by a 10°C pitch, and the vertical axis is the number of pixels having the temperature of each division.

(Maximum Peak Point Detection Step ST3)

In the maximum peak point detection step ST3, the image processor 2 detects a maximum peak point, which is an absolute maximum value of the number of pixels, for each histogram created in the histogram creation step ST2. In the histogram shown in FIG. 3B, a point indicated by a reference symbol P1 is the maximum peak point.

(Intermediate Peak Point Detection Step ST4)

In the intermediate peak point detection step ST4, the image processors 2 detect, for the histogram created in the histogram creation step ST2, an intermediate peak point which has the number of pixels less than the number of pixels of the maximum peak point P1 and is a local maximum value equal to or more than a predetermined threshold value Th of the number of pixels. As shown in FIG. 3B, the threshold value Th of the number of pixels is set to 50% of the number of pixels of the maximum peak point P1. In the histogram shown in FIG. 3B, points indicated by reference symbols P2 become the intermediate peak points.

In addition, the predetermined threshold value Th of the number of pixels is not particularly limited. However, for example, preferably, 50% of the number of pixels of the maximum peak point P1 are set to Th such that a peak of a temperature range considered to be the background such as 1200°C to 1300°C is not ascertained.

(Intermediate Peak Point Counting Step ST5)

In the intermediate peak point counting step ST5, the image processor 2 counts P1, from among the detected intermediate peak points P2, the number Nh of intermediate peak points P2 each having a temperature higher than the temperature at the maximum peak point P1 and the number N1 of intermediate peak points P2 each having a temperature lower than the temperature at the maximum peak point P1. In FIG. 3B, Nh = 1 and N1 = 6.

(Maximum Peak Point Type Determination Step ST6)

In the maximum peak point type determination step ST6, in a case of the number Nh < the number N1 (in a case where the number N1 is larger than the number Nh), the image processor 2 determines that the maximum peak point P1 corresponds to the slag S existing in the molten steel flow F, and in a case of the number Nh > the number N1 (in a case where the number Nh is larger

than the number NI), the image processor 2 determines that the maximum peak point P1 corresponds to the molten steel M existing in the molten steel flow F. In FIG. 3B, since Nh = 1 and N1 = 6, Nh < N1, and thus, it is determined the maximum peak point P1 corresponds to the slag S existing in the molten steel flow F.

(First Determination Step ST7)

In the maximum peak point type determination step ST6, in a case where it is determined that the maximum peak point P1 corresponds to the slag S existing in the molten steel flow F, the image processor 2 performs the first determination step ST7. That is, in the histogram of FIG. 3B, the first determination step ST7 is performed.

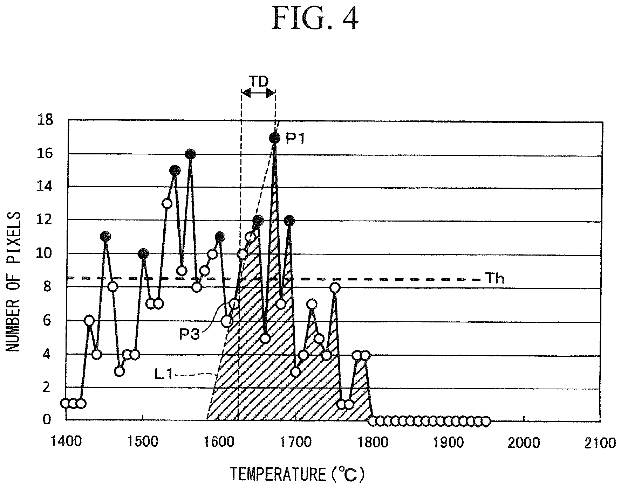

In the first determination step ST7, the image processor 2 determines that, among images constituting the captured image, a pixel having a temperature less than a first threshold value determined based on the maximum peak point P1 corresponds to the molten steel M existing in the molten steel flow F and a pixel having a temperature equal to or more than the first threshold value corresponds the slag S existing in the molten steel flow F. Hereafter, it is described in more detail with reference to FIG. 4 appropriately.

FIG. 4 is a graph for explaining the first threshold value determined in the first determination step ST7. In addition, a histogram shown in FIG. 4 is the same as the histogram shown in FIG. 3B.

As shown in FIG. 4, the first threshold value is represented by a first straight line L1 which passes through the maximum peak point P1 and has a positive inclination in the histogram created in the histogram creation step ST2. Specifically, the first straight line L1 is a straight line which passes through a point P3 and the maximum peak point P1 shown in FIG. 4. The point P3 is the peak point having the highest temperature among from points having the number of pixels less than the threshold value Th of the number of pixels and a temperature less than the temperature at the maximum peak point P1 by a predetermined value TD (for example, 50°C) or more. (that is, the point P3 is a point having the highest temperature among points which have the number of pixels less than a predetermined threshold value Th of the number of pixels the temperature less than the temperature at the maximum peak point P1 by the predetermined value TD or more and are local maximum values).

Here, in a case where it is determined whether or not the point having the highest temperature from among points having the lower temperature by the predetermined value TD or more is a peak point which is a determination target, focusing on a gradient of a line connecting the point and a point adjacent to a low temperature side of the point to each other, if the line has a positive inclination (the line is a line rising to a right), the point of the determination target is regarded as the point P3.

In addition, the slag detection method according to the present embodiment is particularly preferably applied to a molten steel flow in which the number of peaks exceeding the predetermined threshold value Th2 of the number of pixels in the histogram is large. In addition, for example, the slag detection method according to the present embodiment is particularly preferably applied to a molten steel flow in which a peak having 50% or more of the number of pixels of the maximum peak point P1 has three points or more, regardless of a presence or absence of setting of the predetermined threshold value Th of the number of pixels. Characteristics of the peak are determined by a mixing condition between the molten steel and the slag in scouring.

When the temperature of the horizontal axis is defined as X and the number of pixels of the vertical axis is defined as Y, the first threshold value (first straight line L1) is expressed by the following Expression (1).

$$Y = aX + b \ldots (1)$$

Here, a is a positive constant and b is a constant. The constants are determined from the first straight line L1 passing through the point P3 and the maximum peak point P1.

The predetermined value TD is not particularly limited. However, for example, the predetermined value TD is 50°C. In experience, in most cases, a range within $\pm 50°C$ of the maximum peak temperature does not fall on a base. Accordingly, for example, by setting the predetermined value TD to 50°C, preferably, the first threshold value can be determined by the peak excluding the base.

As described above, the image processor 2 determines that the pixel having the temperature less than the first threshold value corresponds to the molten steel M existing in the molten steel flow F. That is, it is determined that the pixel satisfying $Y > aX + b$ corresponds to the molten steel M existing in the molten steel flow F.

Meanwhile, the image processor 2 determines that the pixel having the temperature equal to or more than the first threshold value corresponds to the slag S existing in the molten steel flow F. That is, it is determined that the pixel (pixel corresponding to a hatched region in FIG. 4) satisfying $Y \leq aX + b$ corresponds to the slag S existing in the molten steel flow F.

(Second Determination Step ST8)

In the maximum peak point type determination step ST6, in a case where it is determined that the maximum peak point P1 corresponds to the molten steel M existing in the molten steel flow F, the image processor 2 performs the second determination step ST8.

FIG. 5A is an example of a captured image acquired in the image capturing step ST1 and is a view showing an example different from that of FIG. 3A. Specifically, FIG. 5A shows an another example of the average image obtained by averaging five captured images continuously acquired for each scanning period of the image capturing unit 1.

FIG. 5B is a graph showing a histogram created for the captured image (average image) shown in FIG. 5A. In the histogram of FIG. 5B, in the intermediate peak point counting step ST5, the image processor 2 counts the number Nh of the intermediate peak points P2 each having the temperature higher than the temperature at the maximum peak point P1 to obtain 5 and counts the number of the intermediate peak points P2 each having the temperature lower than the temperature at the maximum peak point P1 to obtain 0. Accordingly, in the subsequent maximum peak point type determination step ST6, since the number Nh > the number N1, the image processor 2 determines that the maximum peak point P1 corresponds to the molten steel M existing in the molten steel flow F.

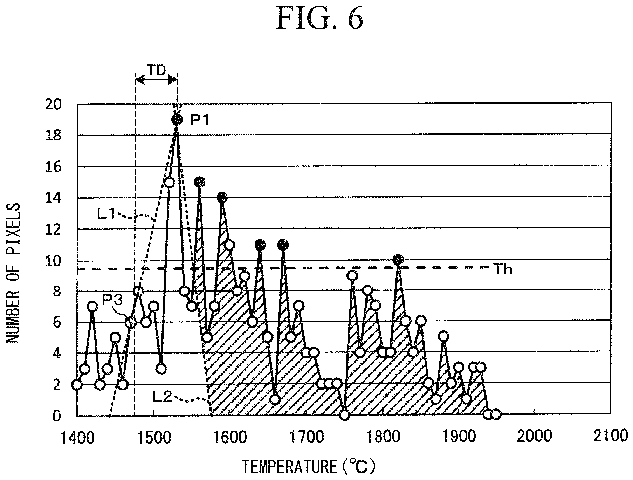

The determination is performed in the maximum peak point type determination step ST6, and thus, the image processor 2 performs the second determination step ST8. In the second determination step ST8, the image processor 2 determines that, among the pixels constituting the captured image, a pixel having a temperature equal to or less than a second threshold value determined based on the maximum peak point P1 corresponds to the molten steel M existing in the molten steel flow F and a pixel having a temperature higher than the second threshold value corresponds the slag S existing in the molten steel flow F. Hereafter, it is described in more detail with reference to FIG. 6 appropriately.

FIG. 6 is a graph for explaining the second threshold value determined in the second determination step ST8. In addition, a histogram shown in FIG. 6 is the same as the histogram shown in FIG. 5B.

As shown in FIG. 6, the second threshold value is represented by a second straight line L2 which passes through the maximum peak point P1 and has a negative inclination. An absolute value of the inclination of the second straight line L2 is larger than an absolute value of the inclination of the first straight line L1 (preferably, the absolute value of the inclination of the second straight line L2 is 1.5 to 2.5 times the absolute value of the inclination of the first straight line L1). The first straight line L1 is the straight line which passes through the point P3 and the maximum peak point P1 shown in FIG. 6. In addition, a gradient of a line between the point P3 and a point adjacent to a low temperature side is positive, and thus, similarly to FIG. 4, the point P3 is a peak point having a highest temperature among from the points having the number of pixels less than the threshold value Th of the number of pixels and a temperature less than the temperature at the maximum peak point P1 by the predetermined value TD (for example, 50°C) or more.

As described above, when the temperature of the horizontal axis is defined as X and the number of pixels of the vertical axis is defined as Y, the first straight line L1 is expressed by the following Expression (1).

$$Y = aX + b \ldots(1)$$

Here, a is a positive constant and b is a constant. The constants are determined from the first straight line L1 passing through the point P3 and the maximum peak point P1.

Meanwhile, for example, if the absolute value of the inclination of the second straight line L2 is set to two times the absolute value of the inclination a of the first straight line L1, the second straight line L2 is expressed by the following Expression (2).

$$Y = -2aX + c \ldots(2)$$

Here, a is a positive constant and c is a constant. In addition, a is determined from the first straight line L1 and c is determined from the second straight line passing through the maximum peak point P1.

As described above, the image processor 2 determines that the pixel having the temperature equal to or less than the second threshold value corresponds to the molten steel M existing in the molten steel flow F. That is, for example, in the histogram shown in FIG. 6, it is determined that the pixel satisfying $Y \leq -2aX + c$ corresponds to the molten steel M existing in the molten steel flow F.

Meanwhile, the image processor 2 determines that the pixel having the temperature higher than the second threshold value corresponds to the slag S existing in the molten steel flow F. That is, for example, in the histogram shown in FIG. 6, it is determined that the pixel (pixel corresponding to a hatched region in FIG. 6) satisfying $Y > -2aX + c$ corresponds to the slag S existing in the molten steel flow F.

According to the slag detection method according to the present embodiment described above, the type of the maximum peak point of the histogram is determined based on a magnitude relationship between the number Nh and Nl of the intermediate peak points in the histogram of the acquired captured image. That is, the type of the maximum peak point is determined without using a fixed threshold value, and thus, even in a case where the temperature of the molten steel flow F is changed, it is possible to accurately determine to which of the molten steel M or the slag S the maximum peak point P1 corresponds.

In addition, the slag detection method according to the present embodiment is premised on a case where the number Nh and the number Nl are different from each other (Nh ≠ Nl). That is, in a case where the number Nh and the number Nl are the same as each other (Nh = Nl), it is not possible to determine the type of the maximum peak point by the above-described method.

Accordingly, for example, in a case where the counted number Nh and number Nl are the same as each other in the intermediate peak point counting step ST5, it is determined to which of the molten steel M or the slag S the maximum peak point P1 corresponds, the following methods (i) and (ii).

(i) Based on an actual temperature and the emissivity of the molten steel flow, a temperature in a case where the maximum peak point P1 corresponds to the molten steel M and a temperature in a case where the maximum peak point P1 corresponds to the slag S are estimated, and the type of the maximum peak point is determined depending on which of the assumed temperatures the temperature at the maximum peak point P1 of the histogram serving as the determination target is closer to.
(ii) Based on that an amount of slag and an amount of molten steel in the converter 3 can be estimated and it is possible to geometrically estimate tilting of the converter 3 which causes the outflow of the molten steel flow F mainly including the molten steel M, it is determined to which of the molten steel M or the slag S the maximum peak point P1 corresponds, from an outflow time of the molten steel flow F.

In addition, according to the slag detection method of the present embodiment, in the first determination step ST7 or the second determination step ST8, it is possible to calculate the number of pixels (area) corresponding to the slag S existing in the molten steel flow F and the number of pixel (area) corresponding to the molten steel M existing in the molten steel flow F. Accordingly, for example, it is possible to an area ratio of the slag S in the molten steel flow F and a volume ratio of the slag S in the molten steel flow F. In addition, if specific gravity of each of the molten steel M and the slag S is used, it is possible to calculate a mass ratio of the slag S in the molten steel flow F, and a flow rate of the molten steel flow F can be estimated from the tilt angle of the converter 3 at the time of tapping. Accordingly, it is possible to estimate an outflow amount (flow rate) of the slag S using the mass ratio of the slag S and the flow rate of the molten steel flow F, and the outflow amount of the slag S can be controlled to a range required in the tapping operation of the converter 3.

Specifically, according to the slag detection method of the present embodiment, the following controls can be performed. That is, the tapping operation ends in a case where the outflow amount or the like (the outflow amount, the number of pixels, the area, the volume, or the like) of the slag S is larger than zero, the tapping operation ends in a case where the outflow amount or the like of the slag S is larger than a predetermined value, the tapping operation ends in a case where a ratio of the outflow amount or the like of the slag S with respect to the outflow amount or the like of the molten steel M is larger than a predetermined value, or the like.

[Example]

Next, Example carried out to confirm operation and effect of the present invention will be described.

The slag detection method according to the present embodiment and the slag detection method described in Patent Document 1 were compared with each other using the captured image shown in FIG. 3A as an evaluation target.

Specifically, in the slag detection method according to the present embodiment, as described above, in the histogram shown in FIG. 3B created for the captured images shown in FIG. 3A, it is determined that the maximum peak point P1 corresponds to the slag S existing in the molten steel flow F. In addition, as shown in FIG. 4, it is determined that the pixels corresponding to the hatched region correspond to the slag S by the first straight line L1 expressed by Expression (1).

In the example shown in FIG. 4, it was determined that 139 pixels correspond to the slag S.

Meanwhile, if the slag detection method disclosed in Patent Document 1 is used, the maximum peak point P1 in the histogram shown in FIG. 3B is considered to correspond to the molten steel M existing in the molten steel flow F. As described above, according to the slag detection method disclosed in Patent Document 1, the pixel having the density value of N1 or more considering the standard deviation $\sigma$ in the horizontal axis direction of the maximum peak point P1 is determined as the molten steel M, and the pixel having the density value of N2 or more obtained by adding the bias value B to the density value N1 is determined as the slag S. If the density value is replaced by a temperature, in the slag detection method described in Patent Document 1, the pixel having a temperature of N1 or more considering the standard deviation $\sigma$ in the horizontal axis direction of the maximum peak point P1 is determined as the molten steel M, and the pixel having a temperature of N2 or more obtained by adding the bias value B to a temperature N1 is determined as the slag S. Here, in the slag detection method described in Patent Document 1, since it is considered that the maximum peak point P1 corresponds to the molten steel M, it is appropriate to set the bias value B to $2\sigma$ or more in order to distinguish the pixel corresponding to the molten steel M and the pixel corresponding to the slag S (That is, the temperature N2 is set to the temperature at the maximum peak point P1 + $\sigma$ or more). In the present evaluation, the minimum value $2\sigma$ at which a detection error of the slag S becomes the smallest is used as the bias value B. Moreover, in the histogram shown in FIG. 3B, assuming that a pixel number distribution having the temperature or more of the maximum peak point P1 is a normal distribution, a value obtained by dividing a sum of the numbers of pixels from the temperature at the maximum peak point P1 to the temperature N2 (the temperature at the maximum peak point P1 + $\sigma$) by a sum of the number of pixels of the temperature or more of the maximum peak point P is about 68%.

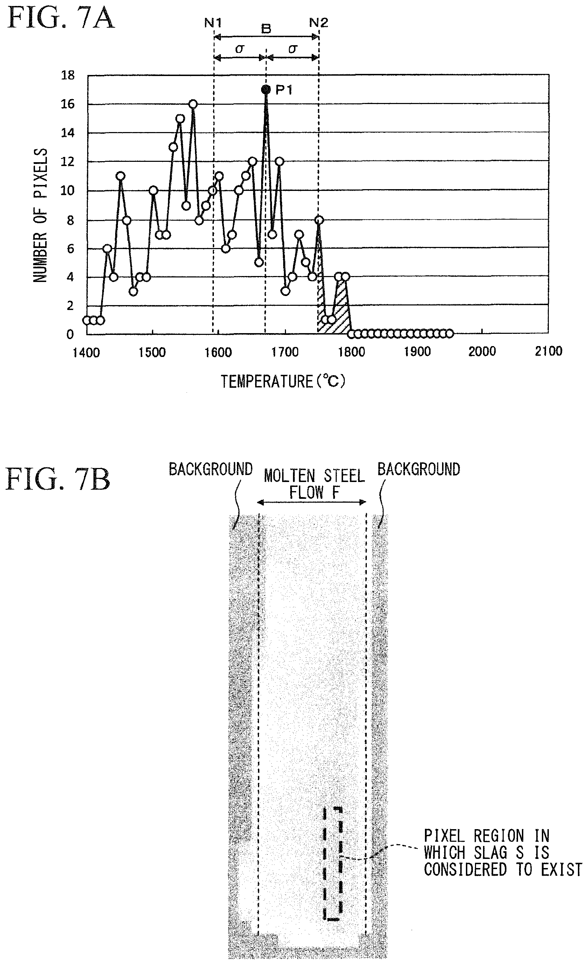

FIGS. 7A and 7B are a graph and a view for explaining the slag detection method described in Patent Document 1. FIG. 7A shows a histogram and FIG. 7B shows a captured image (average image). The histogram shown in FIG. 7A is the same as the histogram shown in FIG. 3B or FIG. 4. The captured image shown in FIG. 7B is the same as the captured image shown in FIG. 3A. According to the slag detection method described in Patent Document 1, it is determined that pixels corresponding to a hatched region in FIG. 7A correspond to the slag S. According to the present evaluation, it was determined that 18 pixels corresponding to a pixel region surrounded by thick dotted lines in FIG. 7B correspond to the slag S.

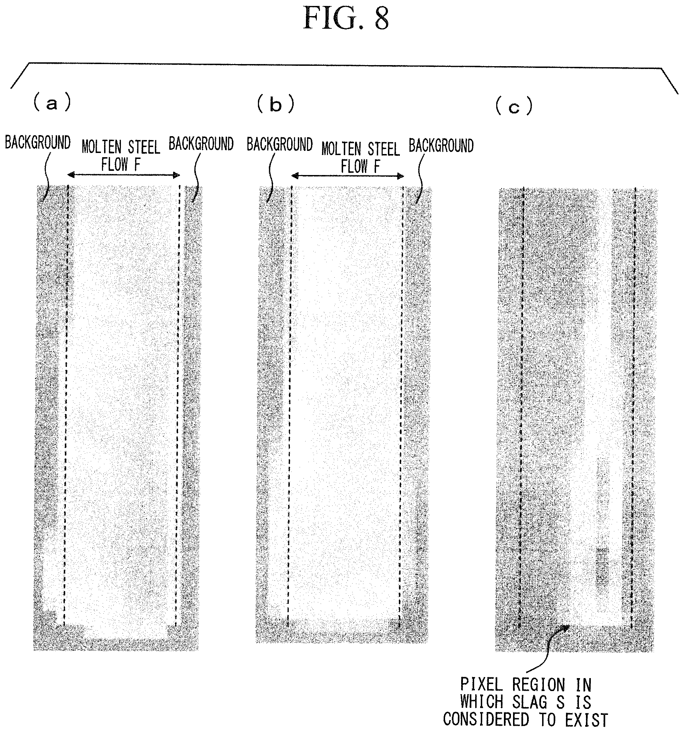

FIG. 8 is a view showing a result of extracting a pixel region in which the slag S is considered to exist in the captured image shown in FIG. 3A by difference processing. In addition, FIG. 8(a) shows the same captured image as that of FIG. 3A, FIG. 8(b) shows the captured image of the molten steel flow in which substantially only the molten steel M exists in the early stage of the tapping, and FIG. 8(c) shows a differential image between the captured image shown in FIG. 8(a) and the captured image shown in FIG. 8(b).

FIG. 8 shows a monochrome display for convenience of illustration. However, in the differential image shown in FIG. 8C, considering that a temperature decreases from a center of a pixel region (a pixel region having a different color (yellow or red) from a color (green) of a pixel region corresponding to a background), which has a temperature higher than that of the pixel region corresponding to the background, toward the vicinity thereof, and considering a form which is elongated vertically with falling of the molten steel flow F, the pixel region having a higher temperature is a pixel region which is considered to have the slag S. The number of pixels of the pixel region (the pixel region colored with yellow or red) having a high temperature was 111.

Accordingly, assuming that 111 estimated as the differential image is a true value of the number of pixels corresponding to the slag S, there were errors of the true value - 83.8% ((18 - 111)/111 x 100 = -83.8) in the slag detection method described in Patent Document 1 while there were errors of the true value + 25.2% ((139-111)/111 x 100 = -25.2) in the slag detection method according to the present embodiment. Therefore, compared to the slag detection method described in Patent Document 1, according to the slag detection method of the present embodiment, it is possible to accurately detect the slag S in the molten steel flow F. This is visually clearer in that, compared to the thick dotted lines shown in FIG. 7B, the thick dotted lines shown in FIG. 3A are closer to an outline of the pixel region shown in FIG. 8(c) in which the slag S is considered to exist.

In addition, if the number of pixels corresponding to the slag S estimated as the differential image is converted into an area (actual size), an area of one pixel is about 9cm$^2$, 9 x 111 = 999 cm$^2$. If this is simply converted into a volume (converted assuming that dimensions of the slag S in a visual axis direction of the image capturing unit 1 are the same as dimensions of the slag S in a visual field plane of the image capturing unit 1), $(999)^{3/2} = 31575\ cm^3 = 31575 \times 10^{-6}\ m^3$. Accordingly, if the specific gravity of the slag S is set to $2 \times 10^{-3}\ m^3/kg$, the mass of the slag S is $(31575 \times 10^{-6})/(2 \times 10^{-3}) = 16\ kg$.

Similarly, if the slag S detected by the slag detection method according to the present embodiment is converted into the mass, the mass is 22 kg (137.5% of the true value), and if the slag S detected by the slag detection method described in Patent Document 1 is converted into the mass, the mass is 1 kg (6.3% of the true value). That is, compared to the method described in Patent Document 1 in which an error of -93.7% occurs, according to the slag detection method of the present embodiment, an error of -37.5% in the mass occurs, and thus, it is possible to accurately detect the slag S in the molten steel flow F.

Hereinbefore, the embodiment of the present invention is described. However, the embodiment is suggested as an example, and the scope of the present invention is not limited to only the embodiment. The embodiment can be embodiment in other various aspects, and various omissions, replacements, and modifications can be made within the scope which does not depart from the gist of the present invention. The embodiments or the modifications thereof are included in the invention described in the claims and the equivalents thereof as well as included in the scope and the gist of the present invention.

For example, in the above-described embodiment, the case is described, in which the first threshold value is represented by the first straight line L1 which passes through the maximum peak point P1 and has a positive inclination. With respect to how to determine the threshold value, in order to detect the slag with higher accuracy, it is preferable to perform the determination by performing fitting with a Gaussian distribution or the like on each of the peaks of the molten steel and the slag. However, this method requires a long calculation time, which is not preferable from the viewpoint of industry. Accordingly, it is possible to more easily determine the threshold value by representing the first threshold value as the straight line.

In addition, the present invention is not limited to the case where the first threshold value and the second threshold value are represented by the first straight line L1 and the second straight line L2. For example, each of the first threshold value and the second threshold value may be represented by a straight line (a straight line with an infinite inclination) orthogonal to the horizontal axis in consideration of the dispersion in the horizontal axis direction of the maximum peak point.

[Brief Description of the Reference Symbols]

| | |
|---|---|
| 1: | image capturing unit |
| 2: | image processor |
| 3: | converter |
| 4: | ladle |
| 100: | slag detection device |
| ST1: | image capturing step |
| ST2: | histogram creation step |
| ST3: | maximum peak point detection step |
| ST4: | intermediate peak point detection step |
| ST5: | intermediate peak point counting step |
| ST6: | maximum peak point type determination step |
| ST7: | first determination step |
| ST8: | second determination step |
| F: | molten steel flow |
| M: | molten steel |
| S: | slag |

## Claims

1. A method of detecting slag in a molten steel flow, comprising:

an image capturing step of sequentially capturing a molten steel flow which is directed from a converter toward a ladle and includes molten steel and slag to acquire a captured image of the molten steel flow;

a histogram creation step of creating, by performing imaing processing on the captured image, a histogram in which a density parameter corresponding to a density of each pixel constituting the captured image is shown on a horizontal axis and the number of pixels that is a total number of pixels each having the density parameter is shown on a vertical axis;

a maximum peak point detection step of detecting a maximum peak point, in which the number of pixels is an absolute maximum value, for the histogram;

an intermediate peak point detection step of detecting an intermediate peak point which has the number of pixels less than the number of pixels of the maximum peak point and is a local maximum value equal to or more than a predetermined threshold value of the number of pixels, for the histogram;

an intermediate peak point counting step of counting the number Nh of intermediate peak points having a density parameter larger than the density parameter at the maximum peak point and the number Nl of intermediate peak points having a density parameter smaller than the density parameter at the maximum peak point; and

a maximum peak point type determination step of determining that the maximum peak point corresponds to the molten steel in a case where the number Nh is larger than the number Nl while determining that the maximum peak point corresponds to the slag in a case where the number Nl is larger than the number Nh.

2.  The method of detecting slag in a molten steel flow according to claim 1, further comprising:

    a first determination step of, in the maximum peak point type determination step, in a case where it is determined that the maximum peak point corresponds to the slag, determining that a pixel having a density parameter less than a first threshold value determined based on the maximum peak point corresponds to the molten steel and a pixel having a density parameter equal to or more than the first threshold value corresponds to the slag; and
    a second determination step of, in the maximum peak point type determination step, in a case where it is determined that the maximum peak point corresponds to the molten steel, determining that a pixel having a density parameter equal to or less than a second threshold value determined based on the maximum peak point corresponds to the molten steel and a pixel having a density parameter more than the second threshold value corresponds to the slag.

3.  The method of detecting slag in a molten steel flow according to claim 2, wherein the first threshold value is represented by a first straight line, which passes through the maximum peak point and has a positive inclination, in the histogram,
    wherein the second threshold value is represented by a second straight line, which passes through the maximum peak point and has a negative inclination, in the histogram, and
    wherein an absolute value of an inclination of the second straight line is larger than an absolute value of an inclination of the first straight line.

4.  The method of detecting slag in a molten steel flow according to claim 3, wherein the first straight line is a straight line which passes through a peak point having a maximum density parameter from among points having the number of pixels less than a threshold value of the number of pixels and a density parameter smaller by a predetermined value or more with respect to the density parameter at the maximum peak point, and the maximum peak point, and wherein the absolute value of the inclination of the second straight line is 1.5 to 2.5 times the absolute value of the inclination of the first straight line.

FIG. 1

FIG. 2

```
              ┌─────────────┐
              │    START    │
              └──────┬──────┘
                     │
                     ▼
        ┌──────────────────────┐
        │ IMAGE CAPTURING STEP │────── ST1
        └──────────┬───────────┘
                   │
                   ▼
        ┌──────────────────────┐
        │  HISTOGRAM CREATION  │────── ST2
        │        STEP          │
        └──────────┬───────────┘
                   │
                   ▼
        ┌──────────────────────┐
        │ MAXIMUM PEAK POINT   │────── ST3
        │   DETECTION STEP     │
        └──────────┬───────────┘
                   │
                   ▼
        ┌──────────────────────┐
        │ INTERMEDIATE PEAK    │────── ST4
        │ POINT DETECTION STEP │
        └──────────┬───────────┘
                   │
                   ▼
        ┌──────────────────────┐
        │ INTERMEDIATE PEAK    │────── ST5
        │ POINT COUNTING STEP  │
        └──────────┬───────────┘
                   │
                   ▼
        ┌──────────────────────┐
        │ MAXIMUM PEAK POINT   │────── ST6
        │ TYPE DETERMINATION   │
        │        STEP          │
        └──┬────────────────┬──┘
```

Nh<Nl
MAXIMUM PEAK POINT
= SLAG

Nh>Nl
MAXIMUM PEAK POINT
= MOLTEN STEEL

```
  ┌──────────────┐      ┌──────────────┐
  │    FIRST     │      │    SECOND    │
ST7│ DETERMINATION│      │ DETERMINATION│── ST8
  │     STEP     │      │     STEP     │
  └──────┬───────┘      └──────┬───────┘
         │                     │
         └──────────┬──────────┘
                    │
                    ▼
              ┌─────────────┐
              │     END     │
              └─────────────┘
```

FIG. 3A

BACKGROUND MOLTEN STEEL BACKGROUND
FLOW F

PIXEL REGION IN
WHICH SLAG S IS
CONSIDERED TO EXIST

FIG. 3B

# FIG. 4

FIG. 5A

BACKGROUND   MOLTEN STEEL   BACKGROUND
                 FLOW F

PIXEL REGION IN
WHICH SLAG S IS
CONSIDERED TO EXIST

FIG. 5B

# FIG. 6

FIG. 7A

FIG. 7B

PIXEL REGION IN WHICH SLAG S IS CONSIDERED TO EXIST

# FIG. 8

(a)  (b)  (c)

BACKGROUND   MOLTEN STEEL   BACKGROUND     BACKGROUND   MOLTEN STEEL   BACKGROUND
                FLOW F                                       FLOW F

PIXEL REGION IN
WHICH SLAG S IS
CONSIDERED TO EXIST

# INTERNATIONAL SEARCH REPORT

| | International application No. |
|---|---|
| | PCT/JP2018/004831 |

### A. CLASSIFICATION OF SUBJECT MATTER
Int.Cl. C21C5/46(2006.01)i, G01N21/17(2006.01)i, G01N33/20(2006.01)i

According to International Patent Classification (IPC) or to both national classification and IPC

### B. FIELDS SEARCHED

Minimum documentation searched (classification system followed by classification symbols)
Int.Cl. C21C5/46, G01N21/17, G01N33/20

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2006-213965 A (JFE STEEL CORPORATION) 17 August 2006, entire text, fig. 1-6 (Family: none) | 1-4 |
| A | JP 2007-246959 A (NIPPON STEEL CORPORATION) 27 September 2007, entire text, fig. 1-11 (Family: none) | 1-4 |
| A | JP 2003-19553 A (NIPPON KOKAN KK) 21 January 2003, entire text, fig. 1-6 (Family: none) | 1-4 |

☐ Further documents are listed in the continuation of Box C.   ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 29.03.2018 | 10.04.2018 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017025440 A
- JP 2006213965 A